# EUROPÄISCHE PATENTANMELDUNG

(11) **EP 0 592 382 A1**
(43) Veröffentlichungstag der Anmeldung: **13.04.1994**
(21) Anmeldenummer: 93890118.8
(22) Anmeldetag: 16.06.1993
(51) Int. Cl.: A61K 35/78, A61K 41/00

(54) **Verfahren zur Gewinnung eines therapeutischen Pflanzenextraktes**

(30) Priorität: 07.10.1992 AT 1972/92
(71) Anmelder: Koch, Heinrich Paul, Dr., A-3400 Klosterneuburg (AT)
(72) Erfinder: Koch, Heinrich Paul, Dr., A-3400 Klosterneuburg (AT)

(57) **Zusammenfassung**

Gegenstand der Erfindung ist ein verbessertes Verfahren zur Gewinnung eines geruchlosen Wirkstoffkomplexes aus Pflanzen der Gattung Allium, vornehmlich aus dem Knoblauch (Allium sativum) und der Küchenzwiebel (Allium cepa). Das erfindungsgemäße Verfahren vereinigt in sich drei Vorteile:
1) Es extrahiert die eigentlichen Wirkstoffe aus der Droge und reichert sie im Extrakt bzw. Lyophilisat an;
2) es verhindert die Bildung und/oder beseitigt die geruchsintensiven Begleitstoffe; und
3) es erzeugt einen lockeren, homogenen, direkt zur galenischen Verarbeitung geeigneten Arzneistoff.

Ein wesentlicher Bestandteil des Verfahrens ist das Tiefgefrieren des Pflanzenmaterials vor der Extraktion mit einem polaren Lösungsmittel, vornehmlich Wasser, mit oder ohne Zusatz von niederen Alkoholen. Zusätzlich kann das Pflanzenmaterial vor dem Tiefgefrieren einer kurzen Mikrowellenbehandlung unterzogen werden, wobei die Bestrahlungsdaner 10 sec und die Bestrahlungsenergie 500 W nicht übersteigen darf. Das Extraktionsgut wird anschließend lyophilisiert und kryopelletiert.

## Beschreibung

Die Erfindung betrifft ein verbessertes Verfahren zur Gewinnung von geruchlosen Wirkstoffkomplexen aus Pflanzen, vornehmlich solchen der Gattung Allium, insbesonders aus dem Knoblauch (Allium sativum L) und der Küchenzwiebel (Allium cepa L.).

Die Lauchgewächse werden in der Volksmedizin seit urdenklichen Zeiten ihrer verschiedenartigen Wirkungen wegen geschätzt. Die moderne phytomedizinische Forschung hat diese Effekte weitgehend bestätigt (Übersich bei H.P. Koch & G.Hahn : Knoblauch - Grundlagen der therapeutischen Anwendung von Allium sativum L., Urban & Schwarzenberg, Munchen 1988).

Ein großer Nachteil ist jedoch der von vielen Menschen als unangenehm empfundene, penetrante Geruch, der den bislang arzneilich verwendeten Zubereitungen aus diesen Pflanzen anhaftet. Alle bisher zur Minderung des Geruchs eingeleiteten Maßnahmen haben sich als nicht zielführend erwiesen (Zusammenfassung bei H.P.Koch, Deutsche Apotheker Zeitung 129 (1989) 1991-1997).

Grundlage der vorliegenden Erfindung ist die Erkenntnis, wonach das eigentliche Wirkprinzip der Laucharten nicht (allein) in den geruchsintensiven Schwefelverbindungen (Allicin, Dialkyloligosulfide, Ajoene, Vinyldithiine, etc.) zu sehen ist, sondern vielmehr bevorzugt in der geruchsarmen bis geruchlosen polaren Fraktion, z.B. von Extrakten, enthalten ist.

Im folgenden wird ein besonders schonendes Verfahren für die Extraktion und Anreicherung der polaren Inhalts- und Wirkstoffe der Allium-Arten unter gleichzeitigem Ausschluß der degenerierten, geruchsintensiven Komponenten beschrieben. Das Endprodukt fällt zugleich in einer Form an, die zur direkten Weiterverarbeitung in für die Phytotherapie passende Darreichungsformen geeignet ist.

Der Heilpflanzenforschung ist heute bekannt, daß die schwefelhaltigen Geruchsträger der Allium-Arten erst im Verlauf der Aufarbeitung des pflanzlichen Ausgangsmaterials aus an und für sich geruchlosen und stabilen Vorläufern gebildet werden. Bei der Verletzung des pflanzlichen Gewebes, z.B. beim Zerkleinern der Zellen und Zellverbände, wird ein Enzym (z.B. Alliinase) aktiviert, unter dessen Einfluß der Prekursor (z.B. Alliin) in einen instabilen, geruchsintensiven Thiosulfinsäureester (z.B. Allicin) umgelagert wird. Dieses Intermediärprodukt wandelt sich spontan in eine Reihe weiterer, widerlich riechender Folgeprodukte um (z.B. Diallyldisulfid), die letztendlich für den typischen Geruch verantwortlich sind. Gelingt es nun, vor der Zerkleinerung und Extraktion, dieses pflanzenimmanente Enzym zu inaktivieren, dann unterbleibt die Bildung der Geruchsträger.

Es wurde nun gefunden, daß eine Behandlung des unverletzten Pflanzenmaterials mit Mikrowellen, z.B. in einem serienmäßigen Mikrowellenherd, das Enzymprotein wirkungsvoll denaturiert. Bei der anschließenden Zerkleinerung und Extraktion mit einem polaren Lösungsmittel (z.B. wässerigem Alkohol) gehen die Wirkstoffe in Lösung, ohne daß nennenswerte Mengen an geruchsaktiven Substanzen gebildet und mitextrahiert werden. Diese Behandlungsmethode ist viel schonender als die bisher übliche nachträgliche Entfernung der flüchtigen Geruchsstoffe, etwa durch Wasserdampfdestillation.

In der europäischen Patentschrift EP A1-0,429.080 (1989/1991) wird die Extraktion mit wäßrigem Alkohol beschrieben, wobei aber NaOH zugesetzt wird, um auf pH 7-12 einzustellen; außerdem wird Cystein zugesetzt, um die instabilen Schwefelverbindungen zu stabilisieren. Als erwünschtes Produkt wird S-Allyl-cystein genannt, dem Wirkstoffcharakter zugeschrieben wird. Es wurde nun gefunden, daß diese Vorgangsweise ungünstig ist, weil diese Schwefelverbindungen nicht dem wirksamen Prinzip der Allium-Arten entsprechen. Der erfindungsgemäße Extrakt, bei dem die Bildung dieser Komponenten ausgeschlossen ist, erscheint dennoch voll wirksam. Ein pH >7 ist bei der Extraktion nicht erforderlich, und ebenso ist die Stabilisierung der S-haltigen Geruchsträger unerwünscht; beides liegt nicht in der Zielsetzung des erfindungsgemäßen Verfahrens.

In der japanischen Patentschrift JP 81,164.762 (1980/1981), Chemical Abstracts 96, 121.210 (1982), wird die Bestrahlung des Knoblauchs mit Mikrowellen der Frequenz 2450 Hz durch 3 bis 4 Perioden von je 30 sec und die anschließende Extraktion mit Ethanol beschrieben. Es wurde nun gefunden, daß diese Vorgangsweise nicht vorteilhaft ist: Zwar werden durch die sehr intensive Bestrahlung die Enzyme restlos inaktiviert, es werden dadurch aber auch die empfindlichen Wirkstoffe weitgehend zerstört bzw. geschädigt.

In systematischen Untersuchungen konnte nachgewiesen werden, daß mit zunehmender Dauer der Mikrowelleneinwirkung die Wirksamkeit der Knoblauchextrakte stetig abnimmt. Empfindliche Bindungen wie Glykosid-, Amid-, Estergruppen u.dgl. werden durch die thermische Belastung gespalten, was eine Wirkungseinbuße nach sich zieht. Weiters wurde gefunden, daß eine kurzfristige Bestrahlung in einem Mikrowellengerät bei 500 W durch maximal 10 sec ebenfalls die Enzyme inaktiviert, was an der verminderten Geruchsentwicklung bei der anschließenden Aufarbeitung deutlich festgestellt werden kann; die Wirksamkeit der so gewonnenen Extrakte erleidet dabei keine Einbuße gegenüber dem bestrahlten Produkt.

Ferner wurde gefunden, daß durch Tieffrieren des Drogenmaterials eine Erhöhung der Ausbeute an wirksamen Inhaltsstoffen erzielt werden kann. So hat die Einlagerung der Knoblauchzehen in einer Tiefkühltruhe bei - 20° C über Nacht vor der weiteren Verarbeitung eine Steigerung der Wirksamkeit bei dem erhaltenen Extrakt zur Folge. Es wird angenommen, daß durch den Kältescbock die Zellstruktur gelockert wird, so daß bei der nachfolgenden Extraktion des homogenisierten Drogenmaterials die löslichen Wirkstoffe leichter aus dem Gewebsverband freigesetzt werden. Zusätzliche Kühlung bei den nachfolgenden Aufbeitungsschritten, etwa durch Außenkühlung mit Eis oder durch Zugabe von Trockeneis zum Extraktionsgut, erweist sich ebenfalls als günstig.

Ein weiteres Problem ist die restlose Entfernung des Lösungsmittels im Anschluß an die Extraktion. Es wurde gefunden, daß durch Abdampfen des organischen Lösungsmittels bei maximal 35 bis 40° C und Gefriertrocknung (Lyophilisation) nicht nur das Vehikel sowie das Pflanzenwasser rückstandslos entfernt, sondern auch Spuren eventuell gebildeter Geruchsträger komplett beseitigt werden. Die wertvollen Extraktstoffe werden bei diesem Verfahren durch die extrem tiefe Temperatur optimal geschont.

Außerdem wurde gefunden, daß durch gleichzeitige Kryopelletierung im Verlauf des Trocknungsprozesses ein lockeres, gut fließfähiges, nicht hygroskopisches Pulver niedriger Dichte entsteht, das sich nach Kompaktierung direkt zu geeigneten festen und damit haltbaren Arzneiformen verarbeiten läßt. Das Produkt kann beispielsweise direkt zu Tabletten bzw. Drageekernen gepreßt oder in Gelatinekapseln gefüllt werden.

Das erfindungsgemäße Verfahren vereinigt somit drei Vorteile: 1) Es extrahiert die eigentlichen Wirkstoffe aus der Droge und reichert sie im Extrakt bzw. Lyophilisat an; 2) es verhindert die Bildung und/oder beseitigt die geruchsintensiven Begleitstoffe; und 3) es erzeugt einen lockeren, homogenen, direkt zur galenischen Verarbeitung geeigneten Arzneistoff.

Die therapeutisch nutzbare Wirkung der Zubereitung läßt sich anhand eines in der Literatur beschriebenen pharmakologischen Tests (H.P.Koch et al., Phytotherapy Research 6 (1991) 50-52) demonstrieren. Hierbei wird die Hemmung des Enzyms Adenosindeaminase (ADA) durch steigende Konzentrationen der Extraktstoffe in vitro geprüft. Es wurde gefimden, daß eine strenge Dosisabhängigkeit des Effektes der erfindungsgemäßen Extrakte aus Knoblauch und anderen Allium-Arten gegenüber ADA besteht, wobei die mittlere inlubitorische Konzentration (IC₅₀) in der Regel bei ca. 10 µg/ml liegt.

### Ausführungsbeispiele

### Beispiel 1

100 g ungeschälte, reife Zehen der Knoblauchzwiebel werden im Tiefkühlschrank über Nacht bei -20° C eingefroren. Das hartgefrorene Drogenmaterial wird anschließend mit 200 g demineralisiertem Wasser im Mixgerät homogenisiert. Der Pfanzenbrei wird 2 Stunden bei Raumtemperatur unter gelegentlichem Umschwenken stehen gelassen und dann abgepreßt. Das klare Filtrat wird anschließend in einer Kryopelletieranlage bis zur Gewichtskonstanz getrocknet. Ausbeute 17 g eines weißen, lockeren Pulvers.

Der Preßrückstand wird nochmals mit 60 ml Methanol und 140 ml Wasser homogenisiert, 2 Stunden stehen gelassen und wieder abgepreßt. Bei der anschließenden Gefriertrocknung werden weitere 3 g pulverförmiger Extrakt erhalten.

### Beispiel 2

Wie bei Beispiel 1, nur daß man vor dem Tieffrieren die Knoblauchzehen in einem Mikrowellengerät mit einer Energie von 500 Watt 5 bis 10 sec lang bestrahlt.

### Beispiel 3

Wie bei Beispiel 1, nur daß die Filtrate vereinigt und gemeinsam hyopelletiert werden.

### Beispiel 4

Wie bei Beispiel 1, nur daß Ethanol anstelle von Methanol verwendet wird.

### Beispiel 5

Wie bei Beispiel 1, nur daß das homogenisierte Pflanzenmaterial direkt mit 200 g Methanol exhahiert wird.

### Beispiel 6

Wie bei Beispiel 1, nur daß das homogenisierte Pflanzenmaterial mit 200 g Isopropanol oder 200 g n-Butanol extrahiert wird.

### Beispiel 7

100 g frische, von den trockenen Häuten befreite Küchenzwiebel werden tiefgefroren und wie bei Beispiel 1 und/oder Beispiel 2 aufgearbeitet. Ausbeute: 3 g Trockenextrakt

## Patentansprüche

1. Verfahren zur Gewinnung eines geruchsarmen aus Pflanzen der Gattung Allium für therapeutische Zwecke durch Extraktion des Pfanzenmaterials mit einem polaren Lösungsmittel, dadurch gekennzeichnet, daß man das rohe Pflanzenmaterial vor der Extraktion bei -20° C tiefgefriert und danach direkt in einem polaren Lösungsmittel homogenisiert und extrahiert und es anschließend durch Abdampfen des organischen Lösungsmittels und Lyophilisieren von dem Lösungsmittel restlos befreit.

2. Verfahren nach Anspruch 1, dad. gek., daß man das Drogenmaterial vor dem Tieffrieren kurzzeitig einer Mikrowellenbehandlung unterzieht, wobei die Bestrahlungsdauer 10 sec nicht überschreitet und die Energie 500 W nicht übersteigt.

3. Verfahren nach Anspruch 1, dad. gek., daß man als Lösungsmittel Wasser und/oder niedere Alkohole verwendet.

4. Verfahren nach Anspruch 1, dad. gek., daß man das Extraktionsgut unter Druck auspreßt und die abgepreßte klare Flüssigkeit in einer Gefriertrocknungsanlage kryopelletiert.

5. Verfahren nach Anspruch 1, dad. gek., daß man das Extraktionsgut unter Druck auspreßt und den flüssigen Anteil kryopelletiert, wobei der Kondensator mit einer Temperatur unter - 100° C und einem Druck unter 0,1 mbar gefahren wird.
